# EUROPÄISCHE PATENTSCHRIFT

(11) **EP 1 879 995 B1**
(45) Veröffentlichungstag und Bekanntmachung des Hinweises auf die Patenterteilung: **04.04.2012**
(21) Anmeldenummer: 06724570.4
(22) Anmeldetag: 26.04.2006
(51) Int. Cl.: C12M 1/20, C12M 1/22

(54) **FERMENTATIONSVERFAHREN UND ANORDNUNG ZU DESSEN DURCHFÜHRUNG**
FERMENTATION METHOD AND APPARATUS FOR ITS IMPLEMENTATION
PROCEDE DE FERMENTATION ET DISPOSITIF ASSOCIE

(30) Priorität: 09.05.2005 DE 102005022045
(43) Veröffentlichungstag der Anmeldung: 23.01.2008
(73) Patentinhaber: RWTH Aachen University, 52062 Aachen (DE)
(72) Erfinder: BÜCHS, Jochen, 52074 Aachen (DE); KLEE, Doris, 52074 Aachen (DE); DITTRICH, Barbara, 52062 Aachen (DE); JEUDE, Markus, 3902 Glis (CH)
(74) Vertreter: Kohlmann, Kai
(86) Internationale Anmeldenummer: PCT/EP2006/003838
(87) Internationale Veröffentlichungsnummer: WO 2006/119867

(56) Entgegenhaltungen:
- EP-A- 1 371 412
- WO-A-99/30823
- DE-A1- 10 036 159
- DE-A1- 10 159 091
- DE-A1- 10 246 446
- DE-A1- 10 312 505
- US-A- 5 922 559
- US-A1- 2001 024 805

## Beschreibung

Die Erfindung betrifft ein Fermentationsverfahren unter Fed-batch Bedingungen mit gezielter Zufütterung von Nährstoffen in die Kulturflüssigkeit mehrerer Reaktionsgefäße, wobei in jedes Reaktionsgefäß die Nährstoffe von jeweils mindestens einem Freisetzungssystem abgegeben werden. Außerdem betrifft die Erfindung eine Anordnung zur Durchführung einer Fermentation mit gezielter Zufütterung von Nährstoffen aus Freisetzungssystemen in die Kulturflüssigkeit mehrerer Reaktionsgefäße.

Für biotechnologische Produktionsprozesse hat sich die Betriebsweise mit Zufütterung von Nährstoffen, die sogenannte Fed-batch-Betriebsweise als besonders vorteilhaft erwiesen. Durch die Zufütterung von Nährstoffen während der Fermentation können Wachstums- und Produktionsphasen gegenüber der reinen Batch-Betriebsweise dramatisch verbessert werden. Das Wachstum der Mikroorganismen hängt stark von der Zusammensetzung der Kulturflüssigkeit, insbesondere der darin enthaltenen Nährstoffe ab. Wenn beispielsweise eine Inhibierung durch Nährstoffüberschuss, zu geringe wasseraktivität oder zu hoher osmotischer Druck vorliegt, eine Katabolitrepression auftritt oder während der Fermentation ein Overflowmechanismus einsetzt, bei dem beispielsweise Lactat, Azetat oder Ethanol ausgeschieden wird, können mit der Batch-Fermentation die optimalen Mikroorganismen und Kulturbedingungen nicht gefunden werden.

Von der Batch-Fermentation unterscheidet sich die Fed-batch-Fermentation in einem konventionellen Bioreaktor dadurch, dass das Reaktionsgefäß zu Begin der Fermentation nur teilweise befüllt ist. Erst im Laufe der Fermentation erreicht das Reaktionsgefäß durch die gezielte Zufütterung von Nähstofflösungen das maximale Füllvolumen. Der Zeitpunkt, zu dem mit der gezielten Zufuhr von Nährstofflösungen begonnen wird, hängt von dem jeweiligen Fermentationsverfahren ab.

Ein Ziel von Fed-batch-Fermentationen besteht in vielen Fällen darin, die Konzentration limitierender Nähstoffe oder eines Precursors im Reaktionsgefäß für einen bestimmten Zeitraum in einem für die biologische Reaktion als vorteilhaft ermittelten niedrigen Konzentrationsbereich zu halten. Ein anderes Ziel der Fed-batch-Fermentation kann darin bestehen, in einer ersten Phase lediglich ein Wachstum der Mikroorganismen hervorzurufen und mit Beginn der gezielten Zufütterung eine Stoffumwandlung zu einem gewünschten Produkt einzuleiten.

Die meist empirische Entwicklung einer Nährstoffzuführung für einen Produktionsprozess in Fed-batch-Betriebsweise erfolgt unter hohem zeitlichen und apparativem Aufwand in sequentiell durchgeführten Experimenten in Laborrührreaktoren. Das klassische Instrument zur Entwicklung von fermentativen Produktionsprozessen in der Biotechnologie ist jedoch die Schüttelkolbentechnik. Hiermit können im parallelen Versuchsansatz die Einflüsse vieler wichtiger Prozessparameter wie beispielsweise Nährstoffspektren, pH-Wert, Temperatur, Spurenelementkonzentrationen parallel untersucht werden.

Um die Schüttelkolbentechnik auch für die Entwicklung von Fed-batch-Prozessen nutzen zu können, wurde die Fed-batch-Schüttelkolbentechnik entwickelt (Chemie Ingenieur Technik (68) 11/96). Hierzu wurde die einfache Schüttelkolbentechnik mit einer dem kleinen Reaktionsvolumen angepassten präzisen Dosiertechnik kombiniert. Die Dosiertechnik besteht aus einer hochpräzisen Kolbenpumpe, die unterschiedliche Nährstoffe mittels eines Mehrwegeventils über Dosierleitungen auf mehrere Schüttelkolben verteilt. Die Steuerung der Nährstoffdosierungen wird von einem Prozessrechner übernommen. Damit lässt sich für jeden einzelnen der parallel betriebenen Schüttelkolben über die Vorgabe eines Dosiermenge-Zeitplans ein individuelles Dosierprofil realisieren. Mit dieser Fed-batch-Schüttelkolbenanlage können bis zu 14 Kolben parallel mit 4 verschiedenen Nährstoffen versorgt werden.

Obwohl die bekannte Fed-batch-Schüttelkolbenanlage eine automatisierte, parallele Entwicklung und Optimierung von Dosierstrategien für Fed-batch-Fermentationen im Labormaßstab ermöglicht, ist wegen des nach wie vor hohen apparativen Aufwandes die Anzahl der parallel zu betreibenden Reaktionsgefäße beschränkt. Ein Screening von zahlreichen (z.B. mehreren hundert) unterschiedlichen Mikroorganismen, die eine optimale Ausbeute an Mikroorganismen und/oder Produkt liefern, ist daher wirtschaftlich kaum durchführbar.

In der Arbeit von Lübbe et al., Appl Microbiol Biotechnol (1985) 22:424-427 "Use of controlled-release polymer to feed ammonium Streptomyces clavuligerus cephalosporin fermentations in shake flasks*"* wird beschrieben, dass Nährstoffe in eine Polymermatrix als Freisetzungssystem eingekapselt werden, das einer Kulturflüssigkeit in einem Schüttelkolben zugegeben wird. Die Freisetzungssysteme liegen als Polymerscheiben mit etwa 33 Gew. % Nährstoffanteil und einem Durchmesser von etwa 9 mm in der Kulturflüssigkeit suspendiert vor und werden von dieser von allen Seiten umspült. Jeder Schüttelkolben enthält zwei scheibenförmige Freisetzungssysteme, die die Nährstoffe langsam freisetzen. Nachteilig ist, dass die Freisetzungssysteme auf der kreisenden Kulturflüssigkeit im Schüttelkolben aufschwimmen können. Damit ist keine definierte Kontaktoberfläche der Freisetzungssysteme mit der Kulturflüssigkeit mehr gegeben und die Freisetzungsrate ist unbestimmt. Außerdem wird der Gasaustausch an der Oberfläche der Kulturflüssigkeit in undefinierter Weise behindert.

Mikrotiterplatten sind aus Kunststoff mit z.B. 24, 48 oder 96 Vertiefungen seit einigen Jahrzehnten bekannt und werden auch für die Kultivierung von Mikroorganismen eingesetzt.

Die DE 103 12 505 A1 offenbart ein Kultivierungssystem zur Gewinnung partikelfreier Reinkulturen von wasserlebenden Kleinst- und Kleinorganismen. Als wasserlebende Klein- oder Kleinstorganismen werden insbesondere Bakterien, Algen, Pilze, Einzeller und kleine Vielzeller verstanden. Das Kultivierungssystem besteht aus zwei übereinander angeordneten Kammern, wobei die untere Kammer als Versorgungskammer mit festem Nährsubstrat gefüllt ist und die obere Kammer eine Kulturkammer darstellt, die als Reservoir partikelfreier Organismensuspensionen dient. Als Nährsubstrate werden beispielsweise Cerealien oder Fleisch genannt. Die Versorgungskammer ist von der Kulturkammer durch eine offenporige physikalische Barriere abgetrennt. Als Barriere kann beispielsweise ein offenporiger Schaumstoff dienen mit einer Schichtdicke von mehreren Millimetern und einer Porengröße, die maximal bei etwa zwei Millimeter liegt. Bei der Kultivierung der Organismen sinken absterbendes Zellmaterial sowie partikuläre Stoffwechselprodukte begünstigt durch die Schwerkraft in die unten liegende Versorgungskammer ab. Umgekehrt wird das partikuläre Nährsubstrat aufgrund der geringen Porenweite des Schaumstoffs in der Versorgungskammer zurückgehalten.

Die US 2001/0024805 A1 offenbart eine Mikrotiterplatte bei der die Nährstoffe in einer Beschichtung in den Mulden der Mikrotiterplatte vorliegen. Die Vorrichtung dient der Kultivierung und Bestimmung der Anzahl von Mikroorganismen in der Nahrungsmittelindustrie, beispielsweise um eine Kontamination von Nahrungsmitteln durch Mikroorganismen zu erfassen. Die Mikrotiterplatte weist hydrophile und hydrophobe Bereiche auf. Die Mulden sind mit einer hydrophilen Beschichtung versehen, während die die Mulden umgebenden Bereiche hydrophob beschichtet sind. Die hydrophilen und hydrophoben Bereiche erlauben es, die Kleinstvolumen der Mulden mit Testvolumen der Kulturflüssigkeit zu befüllen, ohne dass eine wechselseitige Kontamination zwischen den einzelnen Mulden zu befürchten ist.

Ausgehend von diesem Stand der Technik liegt der Erfindung die Aufgabe zugrunde, ein Verfahren der eingangs erwähnten Art zu schaffen, das ein Screening unter Fed-batch Bedingungen erlaubt und die Entwicklung und Optimierung von Dosierstrategien für Fed-batch-Fermentationen mit apparativ geringem Aufwand ermöglicht, wobei definierte Freisetzungsraten erreicht werden und der Gasaustausch an der Oberfläche der Kulturflüssigkeit nicht behindert wird.

Des weiteren soll eine zur Durchführung des Verfahrens geeignete Anordnung vorgeschlagen werden.

Die Aufgabe wird bei einem Verfahren der eingangs erwähnten Art dadurch gelöst, dass die Freisetzungssysteme von einer Diffusionsbarriere gebildet werden, in der die Nährstoffe eingebettet sind, die Freisetzungssysteme an den Innenflächen der Reaktionsgefäße in einem Bereich befestigt werden, der anschließend mit der Kulturflüssigkeit in Kontakt gelangt und dass während der Fermentation ein Energieeintrag in die Kulturflüssigkeit erfolgt.

Die Freisetzungssysteme werden entweder an den Böden oder wänden oder sowohl an Böden und Wänden der Reaktionsgefäße immobilisiert. Abweichend zum Stand der Technik liegt das Freisetzungssystem nicht suspendiert in der Kulturflüssigkeit vor. Die mit der Kulturflüssigkeit in Kontakt gelangende Oberfläche ist daher stets gleich. Dadurch wird eine definierte Freisetzungskinetik der im Freisetzungssystem fixierten Nährstoffe erreicht. Außerdem wird der Gasaustausch mit der Gasphase nicht behindert.

Ein Freisetzungssystem im Sinne der Erfindung stellt insbesondere über einen Zeitraum von minimal einer Stunde bis maximal 3 Wochen eine bestimmte Konzentration eines freizusetzenden Nährstoffes in der Kulturflüssigkeit sicher.

In einer Ausgestaltung des erfindungsgemäßen Verfahrens werden die Nährstoffe mit konstantem, der Fermentation angepassten Massestrom abgegeben. Die konstante Zufuhr kann zu einem gewünschten limitierten biologischen Wachstum der Mikroorganismen führen.

Werden die Nährstoffe indes mit variablem Massestrom vom Freisetzungssystem abgegeben, beispielsweise mit einer exponentiellen Zunahme des Massestroms, ist eine nahezu konstante Nährstoffkonzentration zu erzielen.

In einer weiteren Ausgestaltung des erfindungsgemäßen Verfahrens ist es vorgesehen, dass die Nährstoffe mit zeitlicher Verzögerung nach Beginn der Fermentation abgegeben werden, um die Nährstoffabgabe an eine mögliche Lag-Phase der Mikroorganismen anpassen zu können. Der zeitlich verzögert einsetzende Massestrom der Nährstoffe kann ebenfalls variabel oder konstant sein.

Die Freisetzungsrate der Freisetzungssysteme beträgt 0,1 - 10 g Nährstoff x Kultivierungstag / (Liter Kulturvolumen x Stunde), vorzugsweise zwischen 1 - 2 g Nahrstoff x Kultivierungstag / (Liter Kulturvolumen x Stunde), wenn der Nährstoff die Kohlenstoffquelle ist. Wenn der limitierende Nährstoff eine andere Komponente ist, muss entsprechend dem Bedarf der Mikroorganismen weniger Nährstoff freigesetzt werden.

Der Energieeintrag in die Kulturflüssigkeit kann beispielsweise durch Schütteln der Reaktionsgefäße oder Rühren der enthaltenen Kulturflüssigkeit erfolgen. Das Rühren erfolgt vorzugsweise durch Magnetrührer. Eine weitere Variante ist eine Blasensäule, bei der Luft durch Druck in den unteren Teil der Reaktoren eingeblasen wird.

Eine vorteilhafte Anordnung zur Durchführung einer Fermentation mit gezielter Zufütterung von Nährstoffen aus Freisetzungssystemen in die Kulturflüssigkeit mehrerer Reaktionsgefäße, insbesondere nach einem oder mehreren der Ansprüche 1 bis 7, zeichnet sich dadurch aus,
- dass die Reaktionsgefäße miteinander verbunden sind, wobei die verbundenen Reaktionsgefäße insbesondere als Mikrotiterplatte ausgestaltet sind,
- dass das Freisetzungssystem von einer Diffusionsbarriere gebildet wird, in der die Nährstoffe eingebettet sind und
- dass jedes Freisetzungssystem an einer Innenfläche eines der Reaktionsgefäße in einem Bereich befestigt ist, der mit der Kulturflüssigkeit in Kontakt gelangt.

Die erfindungsgemäße Befestigung der Freisetzungssysteme gewährleistet definierte Freisetzungsraten und den ungestörten Gasaustausch der Kulturflüssigkeiten. Die Befestigung der Freisetzungssysteme kann durch Polymerisieren einer Polymermatrix ausgehend von deren Monomeren im Reaktionsgefäß erfolgen. Weiterhin kann außerhalb der Reaktionsgefäße zunächst eine Folie oder Scheibe als Freisetzungssystem gefertigt werden. Diese wird dann in passende Stücke unterteilt und in die Reaktionsgefäße geklebt oder geklemmt.

Die Verbindung der Reaktionsgefäße zu einer baulichen Einheit, insbesondere als an sich bekannte Mikrotiterplatte, erlaubt die rationelle Durchführung einer Vielzahl paralleler Fermentationen auf engstem Raum. Ein weiterer Vorteil der Verbindung der Reaktionsgefäße besteht darin, dass die Einheit, insbesondere die Mikrotiterplatte von Laborrobotern gehandhabt werden kann und sich so ganze Fermentationsprozesse automatisieren lassen.

Mikrotiterplatten mit integrierten, an den Innenflächen befestigten Freisetzungssystemen, die für die Fed-batch Kultivierung von Mikroorganismen angepasste Freisetzungskinetik besitzen, sollen vorzugsweise für den Einmalgebrauch steril verpackt ausgeführt werden.

Die erfindungsgemäße Anordnung, insbesondere unter Verwendung von Mikrotiterplatten für den Einmalgebrauch in steriler Verpackung, senkt die apparativ bedingten Kosten und den Handhabungsaufwand gegenüber bekannten Fermentationsverfahren mit gezielter Zufütterung von Nährstoffen erheblich. Der Nutzer muss lediglich die Mikrotiterplatte mit integrierten Freisetzungssystemen aus der sterilen Verpackung entnehmen und kann diese sofort für eine Fed-batch Kultivierung von z.B. 96 verschiedenen Kulturen mit definierter Nährstoffzufuhr verwenden.

Die Verwendung von Mikrotiterplatten mit integrierten Freisetzungssystemen unterstützt die Einstellung einer definierten Konzentration bestimmter Nährstoffe in den Testansätzen, ohne dass hierzu eine besondere Mess- und Regelanordnung erforderlich ist.

Dadurch dass die Nährstoffe in der Diffusionsbarriere selbst eingebettet sind, gibt sie die Nährstoffe entsprechend ihrer physikalischen Eigenschaften ab. Das derart gebildete Freisetzungssystem besitzt eine Höhe von 50 µm bis 4 mm, bevorzugt von 200 µm bis 2 mm.

Schließlich kommt insbesondere für eine zeitlich versetzte Abgabe von Nährstoffen ein Freisetzungssystem in Betracht, das von einem in ein Polymer eingebetteten Nährstoffvorrat (Polymermatrix) gebildet wird, wobei der eingebettete Nährstoffvorrat von einem Teil der Innenfläche des Wells und einer mit der Innenfläche des Wells abschließenden Diffusionsbarriere gekapselt ist. Diese Diffusionsbarriere enthält in der Regel keine Nährstoffe, kann aber eventuell auch geringe Mengen an Nährstoffen enthalten, die als Porenbildner wirken können.

Es können Nährstoffkristalle mit breiter Partikelgrößenverteilung in die Polymermatrix eingearbeitet werden. Bevorzugt sind enge Partikelgrößenverteilungen, da dadurch definiertere Freisetzungssysteme erhalten werden. Die Partikelgrößen der Nährstoffkristalle können von 5 µm bis 2 mm betragen, bevorzugt werden Partikelgrößen von 50µm bis 500µm. Des weiteren können Nährstoffpartikel mit definierter unterschiedlicher Partikelgrößenverteilung oder Mischungen von zwei oder mehreren Fraktionen mit jeweils enger Partikelgrößenverteilung verwendet werden. Das Polymerisationsverfahren der Polymermatrix kann so gesteuert werden, dass sich die Nährstoffe mit unterschiedlichen Partikelgrößen in unterschiedlichen Höhen der Freisetzungssysteme anordnen. So kann zum Beispiel durch definiert eingestellte viskosität der Monomerflüssigkeit und durch Ausnutzung der unterschiedlichen Absetzgeschwindigkeit der unterschiedlich großen Partikel während der Polymerisation erreicht werden, dass die großen Nährstoffpartikel absinken und sich darüber in der Polyermatrix die kleineren Nährstoffpartikel anordnen. Weiterhin kann bei Verwendung von Nährstoffkristallen mit einer einzigen engen Partikelgrößenverteilung eine geschichtete Dichteverteilung der Partikel über die Höhe der Polymermatrix erzielt werden. Durch diese speziellen Fertigungsverfahren können vorteilhafte Freisetzungskinetiken eingestellt werden, die an die Bedürfnisse der Mikroorganismen angepasst sind.

Als polymere Materialien für das Freisetzungssystem kommen natürliche und künstliche Polymere in Betracht, insbesondere die nachfolgenden Polymere bzw. Polymergruppen:
Polysaccharide und ihre Derivate, Polysiloxane, Polyacrylsäure und ihre Derivate, Polycarbonate, Polyolefine und ihre Derivate, Polycarbonsäuren und ihre Derivate, Polyether und ihre Derivate, Polyester und ihre Derivate, Polyamine und Amide und ihre Derivate, Polylsulfone und ihre Derivate, Polyurethane, Polyvinyle und ihre Derivate insbesondere Polyvinylalkohole, sowie Copolymere der genannten Polymere und durch Modifizierung erhaltenen Derivate.

Wird beispielsweise Alginat in Kombination mit Chitosan und Polyamid als natürliche Polymere eingesetzt, sind Freisetzungszeiten über 6 Stunden möglich. Mit dem künstlichen Polymer Eudragid, das in Versuchen als eine einen Nährstoffkern umgebende Schicht eingesetzt wurde, konnten Freisetzungszeiten über 12 Stunden erzielt werden. Des weiteren wurden Versuche mit einer Diffusionsbarriere aus Silikon als künstlichem Polymer durchgeführt, in das vor dem Vernetzen Nährstoffkristalle suspendiert wurden. Die Diffusionsbarriere mit derart eingebetteten Nährstoffen konnte eine Freisetzungsdauer von etwa 3-250 Stunden gewährleisten.

In vorteilhafter Ausgestaltung der Erfindung enthält die Diffusionsbarriere Quellkörper, die die Diffusionsbarriere erst durch Quellvorgänge durchlässig machen. Hierdurch lässt sich eine verzögerte Freisetzung bei einer Fermentation mit einer sogenannten Lag-Phase realisieren.

Lösliche Komponenten in der Diffusionsbarriere bilden als Porenbildner allmählich Kanäle, die die Freisetzung der Nährstoffe durch einen sich vergrößernden Massestrom beschleunigen. Durch Auswahl von Komponenten mit unterschiedlicher Löslichkeit lässt sich die Nährstoffabgabe gezielt steuern.

Wenn die Quellkörper ihr Volumen abhängig vom pH-Wert und/oder von der Temperatur der Kulturflüssigkeit vergrößern und/oder sich die löslichen Komponenten bzw. Porenbildner abhängig vom pH-Wert und/oder von der Temperatur der Kulturflüssigkeit auflösen, lässt sich die Nährstoffabgabe abhängig von dem pH-Wert und oder von der Temperatur steuern, beispielsweise indem die Nährstoffabgabe im Falle einer ansäuernden Kulturflüssigkeit erst dann erfolgt, wenn ein bestimmter pH-Wert bzw. eine bestimmte Temperatur erreicht wird.

Nachfolgend wird die Erfindung an Hand der Figuren näher erläutert. Es zeigen
- Figur 1: eine schematische, geschnittene Seitenansicht eines Reaktionsgefäßes einer Mikrotiterplatte zur Durchführung des erfindungsgemäßen Verfahrens, wobei
- Figur 1 A: ein Reaktionsgefäß mit bodenseitigem Freisetzungssystem zeigt,
- Figur 1 B: ein weiteres Reaktionsgefäß mit bodenseitigem Freisetzungssystem zeigt,
- Figur 1 C: ein Reaktionsgefäß mit einem hohlzylindrischen wandseitigen Freisetzungssystemen zeigt sowie
- Figur 1 D: ein Reaktionsgefäß mit einem sich über dessen Wand und Boden erstreckenden Freisetzungssystem zeigt,
- Figur 2: eine schematische, geschnittene Seitenansicht eines Reaktionsgefäßes einer Mikrotiterplatte mit einem gegenüber Fig 1 abweichend aufgebauten Freisetzungssystem, das nicht Gegenstand der vorliegenden Erfindung ist, und
- Figur 3: eine schematische, geschnittene Seitenansicht eines Reaktionsgefäßes einer Mikrotiterplatte mit einem gegenüber Fig 1 und Fig. 2 abweichend aufgebautem Freisetzungssystem.

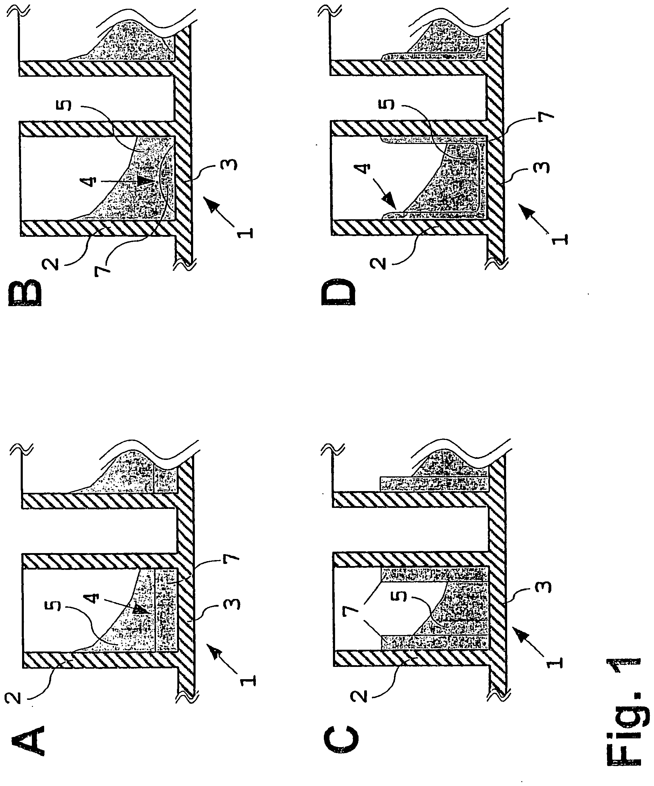

Figur 1 zeigt ein Reaktionsgefäß einer lediglich teilweise dargestellten Mikrotiterplatte, die insgesamt eine Vielzahl derartiger Reaktionsgefäße 1 in gleichmäßiger Anordnung aufweist. Die Reaktionsgefäß 1 einer Mikrotiterplatte werden auch als "well" bezeichnet. Sie sind fest miteinander verbunden und können daher in automatisierten Prozessen einfach gehandhabt werden. Die einzelnen Reaktionsgefäße 1 sind hohlzylindrische, bodenseitig geschlossene Körper und bestehen aus einer umlaufenden Wand 2 und einer Bodenfläche 3.

In den Ausführungsbeispielen nach Figur 1 wird das Freisetzungssystem 4 von einer Diffusionsbarriere 7 aus Polymeren gebildet, in der die Nährstoffe eingebettet sind (Polymermatrix) . Die Einbettung der Nährstoffpartikel wird in Figur 1 durch die weißen Punkte in den Freisetzungssystemen 4 angedeutet.

Nach Figur 1A erstreckt sich das Freisetzungssystem 4 über die gesamte Bodenfläche. Die Befestigung des Freisetzungssystems kann durch Polymerisieren einer Polymermatrix ausgehend von deren Monomeren im Reaktionsgefäß erfolgen.

In Folge der Befestigung der Freisetzungssysteme an den Innenflächen der Reaktionsgefäße, die erst anschließend mit der Kulturflüssigkeit 5 in Kontakt gelangen, lassen sich die Mikrotiterplatten mit an den Innenflächen befestigten Freisetzungssystemen vorproduzieren, steril verpacken und bis zum Einsatzzeitpunkt problemlos lagern.

Die Figuren 1B bis 1D zeigen andere Befestigungsmöglichkeiten des Freisetzungssystems. Figur 1 B zeigt eine Variante, bei der sich das Freisetzungssystem 4 nicht über die gesamte Bodenfläche 3 erstreckt, bei der Variante nach Figur 1C ist ein hohlzylindrisches Freisetzungssystem 4 an der Seitenwand 2 des Reaktionsgefäßes 1 befestigt, während Figur 1D ein die Seitenwand 2 und Bodenfläche 3 überspannendes Freisetzungssystem 4 zeigt.

Weiter verdeutlichen die Figuren 1A bis 1D, dass die Freisetzungssysteme 4 sämtlich in einem Bereich an den Innenflächen des Reaktionsgefäßes 1 befestigt sind, der mit der anschließend einzufüllenden Kulturflüssigkeit 5 in Kontakt gelangt.

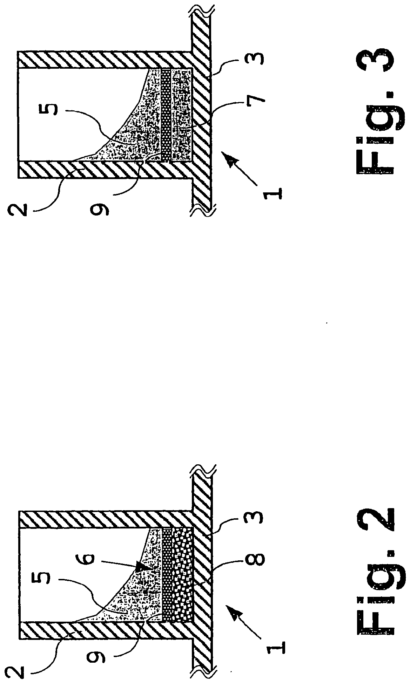

Das Freisetzungssystem 6 nach Figur 2 unterscheidet sich von dem Freisetzungssystem nach Figur 1 dadurch, dass das Freisetzungssystem 6 aus einem Nährstoffvorrat 8 gebildet wird, der von einem Teil der Innenfläche des Wells, nämlich dem Boden 3 und dem unteren Teil der Seitenwand 2 und einer mit der Seitenwand 2 des Wells bündig abschließenden Diffusionsbarriere 9 eingeschlossen ist. Die als Diffusionsbarriere 9 fungierende Polymerschicht deckt den Nährstoffvorrat 8 auf diese Weise ab, so dass er nur durch die Diffusionsbarriere 9 in die Kulturflüssigkeit 5 gelangen kann.

Schließlich kommt insbesondere für eine zeitlich versetzte Abgabe von Nährstoffen ein Freisetzungssystem nach Figur 3 in Betracht, das von einem in ein Polymer eingebetteten Nährstoffvorrat (Polymermatrix) 7 gebildet wird, wobei der eingebettete Nährstoffvorrat (Polymermatrix) 7 von einem Teil der Innenfläche des Wells und einer mit der Innenfläche des Wells abschließenden Diffusionsbarriere 9 gekapselt ist. Die Diffusionsbarriere 9 enthält, wie auch die in Figur 2 dargestellte Diffusionsbarriere 9, keine Nährstoffe.

### Bezugszeichenliste

| Nr. | Bezeichnung |
|---|---|
| 1. | Reaktionsgefäß |
| 2. | Wand |
| 3. | Bodenfläche |
| 4. | Freisetzungssystem |
| 5. | Kulturflüssigkeit |
| 6. | Freisetzungssystem |
| 7. | Diffusionsbarriere (Polymermatrix mit Nährstoffpartikeln) |
| 8. | Nährstoffvorrat |
| 9. | Diffusionsbarriere (Polymerschicht ohne Nährstoffpartikel) |

## Patentansprüche

1. Fermentationsverfahren unter Fed-batch Bedingungen mit gezielter Zufütterung von Nährstoffen in die Kulturflüssigkeit mehrerer Reaktionsgefäße, wobei in jedes Reaktionsgefäß die Nährstoffe von jeweils mindestens einem Freisetzungssystem abgegeben werden, **dadurch gekennzeichnet, dass** die Freisetzungssysteme (4) von einer Diffusionsbarriere (7) gebildet werden, in der die Nährstoffe eingebettet sind, die Freisetzungssysteme (4) an den Innenflächen (2,3) der Reaktionsgefäße (1) in einem Bereich befestigt werden, der anschließend mit der Kulturflüssigkeit (5) in Kontakt gelangt und dass während der Fermentation ein Energieeintrag in die Kulturflüssigkeit erfolgt.

2. Fermentationsverfahren nach Anspruch 1, **dadurch gekennzeichnet, dass** die Freisetzungssysteme (4) an den Böden (3) und/oder Wänden (2) der Reaktionsgefäße (1) befestigt werden.

3. Fermentationsverfahren nach Anspruch 1 oder 2, **dadurch gekennzeichnet, dass** die Nährstoffe mit zeitlicher Verzögerung nach Beginn der Fermentation abgegeben werden.

4. Fermentationsverfahren nach einem der Ansprüche 1 bis 3, **dadurch gekennzeichnet, dass** die Nährstoffe von den Freisetzungssystemen (4) mit konstantem Massestrom abgegeben werden.

5. Fermentationsverfahren nach einem der Ansprüche 1 bis 3, **dadurch gekennzeichnet, dass** die Nährstoffe von den Freisetzungssystemen (4) mit variablem Massestrom abgegeben werden.

6. Fermentationsverfahren nach einem der Ansprüche 1 bis 5, **dadurch gekennzeichnet, dass** die Reaktionsgefäße (1) während der Fermentation geschüttelt werden.

7. Fermentationsverfahren nach einem der Ansprüche 1 bis 5, **dadurch gekennzeichnet, dass** die Kulturflüssigkeit (5) in den Reaktionsgefäßen (1) während der Fermentation gerührt wird.

8. Anordnung zur Durchführung einer Fermentation unter Fed-batch Bedingungen mit gezielter Zufütterung von Nährstoffen aus Freisetzungssystemen in die Kulturflüssigkeit mehrerer Reaktionsgefäße, insbesondere nach einem oder mehreren der Ansprüche 1 bis 7, **dadurch gekennzeichnet,**
- **dass** die Reaktionsgefäße (1) miteinander verbunden sind,
- **dass** sich in jedem Reaktionsgefäß (1) mindestens ein Freisetzungssystem (4) befindet,
- **dass** das Freisetzungssystem (4) von einer Diffusionsbarriere (7) gebildet wird, in der die Nährstoffe eingebettet sind und
- **dass** jedes Freisetzungssystem (4) an einer Innenfläche (2,3) eines der Reaktionsgefäße (1) in einem Bereich befestigt ist, der mit der Kulturflüssigkeit (5) in Kontakt gelangt.

9. Anordnung nach Anspruch 8, **dadurch gekennzeichnet, dass** die verbundenen Reaktionsgefäße (1) als Mikrotiterplatte ausgestaltet sind.

10. Anordnung nach Anspruch 9, **dadurch gekennzeichnet, dass** die Freisetzungssysteme (4) am Boden (3) und/oder der Innenwand (2) der Wells der Mikrotiterplatte befestigt sind.

11. Anordnung nach einem der Ansprüche 8 - 10, **dadurch gekennzeichnet, dass** das Freisetzungssystem (4) von einem Teil der Innenfläche (2,3) des Wells (1) und einer mit der Innenfläche des Wells abschließenden weiteren Diffusionsbarriere (9) gekapselt ist.

12. Anordnung nach einem der Ansprüche 8- 11, **dadurch gekennzeichnet, dass** die Diffusionsbarriere (7,9) zumindest teilweise aus Polymeren besteht.

13. Anordnung nach einem der Ansprüche 8 bis 12, **dadurch gekennzeichnet, dass** die Diffusionsbarriere (7,9) Quellkörper und/oder lösliche Komponenten, die als Porenbildner wirken, enthält.

14. Anordnung nach Anspruch 13, **dadurch gekennzeichnet, dass** die Quellkörper abhängig vom pH-Wert und/oder der Temperatur der Kulturflüssigkeit ihr Volumen vergrößern.

15. Anordnung nach Anspruch 13, **dadurch gekennzeichnet, dass** sich die löslichen Komponenten, die als Porenbildner wirken, abhängig vom pH-Wert und/oder der Temperatur der Kulturflüssigkeit (5) auflösen.

## Claims

1. A fermentation method under fed-batch conditions with controlled feeding of nutrients into the culture liquid of a plurality of reaction vessels, wherein in each reaction vessel the nutrients are released from at least one release system in each case, **characterised in that** the release systems (4) are formed by a diffusion barrier (7) in which the nutrients are embedded, the release systems (4) are attached to the inner surfaces (2, 3) of the reaction vessel (1) in a region which then comes in contact with the culture liquid (5) and that during the fermentation energy is input into the culture liquid.

2. The fermentation method according to claim 1, **characterised in that** the release systems (4) are attached to the bottoms (3) and/or the walls (2) of the reaction vessels (1).

3. The fermentation method according to claim 1 or 2, **characterised in that** the nutrients are released with a time delay after the beginning of fermentation.

4. The fermentation method according to any one of claims 1 to 3, **characterised in that** the nutrients are released by the release systems (4) with constant mass flow.

5. The fermentation method according to any one of claims 1 to 3, **characterised in that** the nutrients are released by the release systems (4) with variable mass flow.

6. The fermentation method according to any one of claims 1 to 5, **characterised in that** the reaction vessels (1) are shaken during fermentation.

7. The fermentation method according to any one of claims 1 to 5, **characterised in that** the culture liquid (5) in the reaction vessels (1) is stirred during fermentation.

8. An arrangement for carrying out a fermentation under fed-batch conditions with controlled feeding of nutrients from release systems into the culture liquid of a plurality of reaction vessels, in particular according to one or more of claims 1 to 7, **characterised in**
- **that** the reaction vessels (1) are connected to each other,
- **that** at least one release system (4) is located in each reaction vessel (1),
- **that** the release system (4) is formed by a diffusion barrier (7) in which the nutrients are embedded and
- **that** each release system (4) is attached to an inner surface (2, 3) of one of the reaction vessels (1) in an area which comes in contact with the culture liquid (5).

9. The arrangement according to claim 8, **characterised in that** the connected reaction vessels (1) are configured as a microtitre plate.

10. The arrangement according to claim 9, **characterised in that** the release systems (4) are attached to the bottom (3) and/or the inner wall (2) of the wells of the microtitre plate.

11. The arrangement according to any one of claims 8-10, **characterised in that** the release system (4) is encapsulated by a part of the inner surface (2, 3) of the well (1) and another diffusion barrier (9) which terminates with the inner surface of the well.

12. The arrangement according to any one of claims 8-11, **characterised in that** the diffusion barrier (7, 9) consists at least partially of polymers.

13. The arrangement according to any one of claims 8 to 12, **characterised in that** the diffusion barrier (7, 9) contains swelling bodies and/or soluble components which act as pore forming agents.

14. The arrangement according to claim 13, **characterised in that** the swelling bodies increase in volume as a function of the pH and/or temperature of the culture liquid.

15. The arrangement according to claim 13, **characterised in that** the soluble components which act as pore forming agents dissolve as a function of the pH and/or the temperature of the culture liquid (5).

## Revendications

1. Procédé de fermentation dans des conditions Fed-batch avec arrivée ciblée de produits nutritifs dans le liquide de culture de plusieurs récipients de réaction, les produits nutritifs étant délivrés dans chaque récipient de réaction par respectivement au moins un système de libération, **caractérisé en ce que** les systèmes de libération (4) sont formés par une barrière de diffusion (7), dans laquelle les produits nutritifs sont insérés, les systèmes de libération (4) étant fixés sur les surfaces intérieures (2, 3) des récipients de réaction (1) dans une zone qui vient en contact ensuite avec le liquide de culture (5) et **en ce qu'**un apport d'énergie dans le liquide de culture intervient pendant la fermentation,

2. Procédé de fermentation selon la revendication 1, **caractérisé en ce que** les systèmes de libération (4) sont fixés sur les fonds (3) et/ou les parois (2) des récipients de réaction (1).

3. Procédé de fermentation selon la revendication 1 ou 2, **caractérisé en ce que** les produits nutritifs sont délivrés avec un retard dans le temps après le début de la fermentation.

4. Procédé de fermentation selon l'une des revendications 1 à 3, **caractérisé en ce que** les produits nutritifs sont délivrés par les systèmes de libération (4) avec un flux de masse constant.

5. Procédé de fermentation selon l'une des revendications 1 à 3, **caractérisé en ce que** les produits nutritifs sont délivrés par les systèmes de libération (4) avec un flux de masse variable.

6. Procédé de fermentation selon l'une des revendications 1 à 5, **caractérisé en ce que** les récipients de réaction (-) sont secoués pendant la fermentation.

7. Procédé de fermentation selon l'une des revendications 1 à 5, **caractérisé en ce que** le liquide de culture (5) est agité dans les récipients de réaction (1) pendant la fermentation.

8. Agencement pour mettre en oeuvre une fermentation dans des conditions Fed-batch avec arrivée ciblée de produits nutritifs provenant de systèmes de libération dans le liquide de culture de plusieurs récipients de réaction, en particulier selon une ou plusieurs des revendication 1 à 7, **caractérisé**
- **en ce que** les récipients de réaction (1) sont reliés les uns aux autres,
- **en ce qu'**au moins un système de libération (4) se trouve dans chaque récipient de réaction (1),
- **en ce que** le système de libération (4) est formé par une barrière de diffusion (7) dans laquelle les produits nutritifs sont insérés et
- **en ce que** chaque système de libération (4) est fixé sur une surface intérieure (2, 3) de l'un des récipients de réaction (1) dans une zone qui vient en contact avec le liquide de culture (5).

9. Agencement selon la revendication 8, **caractérisé en ce que** les récipients de réaction reliés (1) sont conçus sous forme de plaque de microtritrage.

10. Agencement selon la revendication 9, **caractérisé en ce que** les systèmes de libération (4) sont fixés sur le fond (3) et/ou la paroi intérieure (2) des wells de la plaque de microtritrage.

11. Agencement selon l'une des revendications 8 à 10, **caractérisé en ce que** le système de libération (4) est encapsulé par une partie de la surface intérieure (2, 3) du wells (1) et une autre barrière de diffusion (9) se terminant avec la surface intérieure du wells.

12. Agencement selon l'une des revendications 8 à 11, **caractérisé en ce que** la barrière de diffusion (7, 9) est au moins en partie à base de polymères.

13. Agencement selon l'une des revendications 8 à 12, **caractérisé en ce que** la barrière de diffusion (7, 9) contient des corps de gonflement et/ou des composants solubles qui agissent comme des agents porogènes.

14. Agencement selon la revendication 13, **caractérisé en ce que** les corps de gonflement augmentent leur volume en fonction du pH et/ou de la température du liquide de culture.

15. Agencement selon la revendication 13, **caractérisé en ce que** les composants solubles, qui agissent comme des agents porogènes, se dissolvent en fonction du pH et/ou de la température du liquide de culture (5).
